# EUROPEAN PATENT SPECIFICATION

(11) **EP 3 588 378 B1**
(45) Date of publication and mention of the grant of the patent: **30.03.2022**
(21) Application number: 19155713.1
(22) Date of filing: 06.02.2019
(51) Int. Cl.: A61B 5/02, G06K 9/32, G06T 7/00, G06K 9/46, G06K 9/62, A61B 5/00, A61B 6/03, A61B 6/00

(54) **METHOD FOR DETERMINING AT LEAST ONE ENHANCED OBJECT FEATURE OF AN OBJECT OF INTEREST**
VERFAHREN ZUR BESTIMMUNG VON MINDESTENS EINEM OBJEKTMERKMAL EINES OBJEKTS VON INTERESSE
PROCÉDÉ POUR DÉTERMINER AU MOINS UNE CARACTÉRISTIQUE AMÉLIORÉE D'UN OBJET D'INTÉRÊT

(30) Priority: 29.06.2018 EP 18180887
(43) Date of publication of application: 01.01.2020
(73) Proprietor: Siemens Healthcare GmbH, 91052 Erlangen (DE)
(72) Inventor: Wels, Michael, 96047 Bamberg (DE); Mühlberg, Alexander, 90409 Nürnberg (DE); Lades, Felix, 90427 Nürnberg (DE); Sühling, Michael, 91052 Erlangen (DE)

(56) References cited:
- WO-A1-2018/017355
- US-A1- 2016 203 599
- US-A1- 2017 035 381
- TATE JESS ET AL: "Verification of a defibrillation simulation using internal electric fields in a human shaped phantom", COMPUTING IN CARDIOLOGY, N/A, 7 September 2014 (2014-09-07), pages 689-692, XP032737146, ISSN: 2325-8861 ISBN: 978-1-4799-0884-4 [retrieved on 2015-02-17]
- SERMESANT MAXIME ET AL: "Personalised Electromechanical Model of the Heart for the Prediction of the Acute Effects of Cardiac Resynchronisation Therapy", 3 June 2009 (2009-06-03), INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 239 - 248, XP047377029, ISBN: 978-3-642-17318-9 * Section 3.2 *
- CAOUETTE CHRISTIANE ET AL: "Geometry reconstruction method for patient-specific finite element models for the assessment of tibia fracture risk in osteogenesis imperfecta", MEDICAL AND BIOLOGICAL ENGINEERING AND COMPUTING, SPRINGER, HEILDELBERG, DE, vol. 55, no. 4, 17 June 2016 (2016-06-17), pages 549-560, XP036189466, ISSN: 0140-0118, DOI: 10.1007/S11517-016-1526-5 [retrieved on 2016-06-17]
- BO WANG ET AL: "In vivo Intravascular Ultrasound-guided Photoacoustic Imaging of Lipid in Plaques Using an Animal Model of Atherosclerosis", ULTRASOUND IN MEDICINE AND BIOLOGY., vol. 38, no. 12, 1 December 2012 (2012-12-01), pages 2098-2103, XP55608311, US ISSN: 0301-5629, DOI: 10.1016/j.ultrasmedbio.2012.08.006

## Description

The invention concerns a method for determining at least one enhanced object feature of an object of interest, a system comprising a processing unit, a computer program and a computer-readable storage medium.

The invention especially relates to a method for determining at least one enhanced object feature of an object of interest that is at least partially depicted by an image in a region of an anatomy of a patient, said image having been provided from an imaging device, wherein radiomics from local and global features are used.

An automatic extraction of features of an at least partially depicted object is especially useful in medical imaging. Automatic feature extraction is e.g. often used in radiomics. A multitude of images can be analysed to extract the respective features of depicted objects and these features can be stored in a data base. Once a new medical image is analysed and its features are extracted, a resulting feature vector can be used to extract similar cases from the data base. Preferably the different data sets in the data base can be classified according to certain criteria and/or enriched with additional information, e.g. a prognosis and/or development of a certain disease, information concerning therapies, etc.

Radiomics was first used in the area of oncology and used to process computed tomography images. The technique can however be applied to a multitude of medical areas or other areas of research to recognise certain conditions in two dimensional or three dimensional images and/or to provide high level statistical information e.g. to group patients into certain groups, generate a prediction for a survival time or generate information about the probability of certain diseases.

A framework for performing radiomics is discussed in the article van Griethuysen et al., Computational radiomics system to decode the radiographic phenotype, Cancer Research 77(21), November 2017.

Various approaches for using radiomics in the context of Coronary Computed Tomography Angiography (CCTA) are discussed in the articles: Kolossvary et al., Cardiac computed tomography radiomics: a comprehensive review on
radiomic techniques, Journal of Thoracic Imaging 33(1), January 2018; Kolossvary et al., Radiomic features of high risk coronary atherosclerotic plaques in coronary
CT angiography, European Heart Journal, Volume 38, Issue suppl_1, 2017; and Maurovich-Horvat et al., Comprehensive plaque assessment by coronary CT angiography, Nature Reviews Cardiology 11(7), July 2014.

The document US 2016 0203599 A1 discloses a system and method for analyzing quantitative information obtained from radiological images.

Coronary heart disease (CHD) is the most frequent cause of death in the United States with approximately 600,000 deaths every year. For diagnosis, it is possible to use CCTA. For early stages of coronary artery disease the clinician needs to decide whether identifiable atherosclerotic vessel sections ("plaque sections") are at the risk of rupturing and causing potentially life-threatening major adverse cardiac events (MACE). Traditional biomarkers contributing to this decision are often qualitative morphological characteristics like the presence and degree of high risk plaque features (napkin-ring sign, spotty calcification, etc.). The field of radiomics may play a crucial role when it comes to quantifying such characteristics in an automated fashion and to additionally reveal other quantitative biomarkers of a similar kind (texture features) potentially better predicting MACEs from CCTA. Radiomics is an emerging discipline that mines image data to explore novel biomarkers.

However, the perspective in current radiomics, e.g. scientific cardiac radiomics, is restricted to the local and classical feature-based analysis of an object of interest, e.g. diseased vessel sections themselves, without keeping in mind global characteristics that may also have an impact on vulnerability and severity of an associated event like the position of the plaque section in the context of the whole coronary tree. Unlike the Agatston score, that provides global risk stratification from native cardiac CT scans, these restricted approaches fail to provide a comparable global assessment of a patient's disease.

In other words radiomics is focused on information that can be extracted from the object of interest itself, e.g. the diseased tissue or structure, or lesion, such as an artheriosclerotic plaque, a tumor, a bone fracture or the like. However, additional information outside of the region of the object of interest yields additional valuable information regarding the object of interest itself.

Focusing merely on this region of interest (ROI) yielding local features bears the risk to neglect more global features that are highly relevant to the development of the disease: stenosis caused by a sudden rupture of a proximal plaque section will affect the blood supply to the heart muscle much more than in the case of a distal plaque section. Naive lesion-centric approaches may miss the impact of such obvious and important imaging biomarkers because these are only assessable on a semantically higher level of geometric abstraction (lesion-centric vs. whole coronary tree comprising perspective), which is not part of the system's way of looking at things by design.

The object of the present invention is therefore to provide a method for determining at least one feature of an object that can overcome the mentioned problems of the discussed approaches based on local bio markers.

The problem is solved by the method of claim 1 for determining at least one enhanced object feature of an object of interest that is at least partially depicted by an image in a region of an anatomy of a patient, said image having been provided from an imaging device,
wherein a respective object feature for each object and at least one semantic feature are determined from the image data of the image,
wherein said object feature depends on information comprised in image data of a first region of interest, said first region of interest containing the object region, and the semantic feature depends on information comprised in image data of a second region, said second region of interest comprising a region excluding the object region, and wherein said semantic feature comprises an anatomic context information,
wherein the enhanced object feature is determined from the object feature and the semantic feature, wherein the enhanced object feature is determined from the object feature and the semantic feature by processing image data of the first region of interest based on the semantic feature to obtain a processed image data set comprising the first region of interest and determining the enhanced object feature from the processed image data set, and wherein the image data in the first region of interest depends on a patient specific geometry that is described by the semantic feature, wherein the processed image data set is obtained by a transformation of the first region of interest that maps the patient specific geometry to a given standardized geometric space.

The present invention therefore proposes a more holistic approach that allows incorporating global features in addition to the local features in the processing pipeline. The global features that can be described by the semantic feature can e.g. be the position of the object of interest in a standardized frame of reference, a patient specific geometry in the area of the object, etc. The object feature can especially be a local feature, e.g. a biomarker or some other feature extracted from local image data, e.g. a local texture, contrast variation or any of a multitude of local features that are used in radiomics, automated image analysis etc.

The radiomics approaches discussed in the introduction only take these local features into account. The method according to the present invention additionally takes into account the semantic feature that can especially be a more global feature. The inventive method therefore allows for an improved prediction of a property and/or a further behaviour of the imaged object. If e.g. a plaque in a vessel is investigated a risk of rupturing or a major adverse cardiac event or some other information can be provided as enhanced object feature. The quality of prediction can be noticeably improved beyond an accuracy that could be reached by only taking local features into account.

Different ways of using the semantic feature will be discussed in a detail below. An algorithm can then be used to combine this object feature or multiple of these object features with at least one semantic feature to generate information that can e.g. estimate a clinical relevance of an object, predict potential risks to the patient caused by the object, etc. This can be referred to as "explicit use of global image semantics".

The object feature is the image data in the first region of interest and the semantic feature can be directly used to modify or pre-process the image data before higher level features are extracted. In this case the enhanced object feature can either be the pre-processed or modified image data or a feature extracted from this data. Obviously, both of these approaches can be combined. For example the semantic feature can first be used to pre-process the image data and then a feature extracted from this pre-processed image data can be used in conjunction with the same or a different semantic feature to generate an enhanced object feature.

The object of interest may be any object of interest within the patient's anatomy, e.g. an organ, tumor, pathogenic lesion, plaque, etc.

The object region can be the region of the image that depicts the object or that depicts part of the object. The object region can be identical to the first region of interest or it can be part of the first region of interest. The first and second region of interest can also be called first or second region. The second and first region of interest can be distinct or they can overlap or the first region of interest can be within the second region of interest or vice versa. Preferably the second region of interest has a larger area than the first region of interest to allow for an extraction of global information, e.g. a patient specific geometry, as the semantic feature.

An object feature is an information or comprises data regarding the object of interest itself, such as size, texture, CT number (in Hounsfield Units), etc. The object feature can be thought of in terms of a radiomics biomarker related to the object of interest itself. This information can be extracted from the image data in the region of the object of interest itself.

In general the object feature can be any feature, especially a feature vector, determined from the first region of interest. In the simplest case the object feature can be a feature vector comprising all image data of the first region of interest, e.g. all voxel values of the first region of interest. It is also possible to determine the object feature or individual entries of a feature vector forming the object feature by applying an algorithm that can e.g. be previously trained by machine learning, to the image data of the first region of interest. The object feature can depend exclusively on the image data in the first region of interest. Especially when the image data in this region is directly used as the object feature it can be advantageous to first determine an intermediate feature that depends on the object feature and then determine the enhanced object feature based on the intermediate feature.

The determination of an enhanced feature can serve to provide diagnostically relevant information that can e.g. be used to support a medical professional in forming a diagnosis.

A semantic feature or global semantic feature is a feature based at least in part on information in the image data outside of the region of the object of interest itself. It may contain information about the local environment in which the object of interest is present. If, for example, the object of interest is a plaque lesion in a coronary artery the semantic information may comprise information about the relative location of the plaque lesion within the local environment, e.g. the coronary artery tree. In a further example, the semantic information may comprise information about the relative distance to another location or a further object in the patients anatomy, e.g. a distance of a plaque lesion to the next upstream or down-stream artery bifurcation, a distance of a plaque lesion to the aortic root of the affected coronary artery, a distance of a tumor to the closest major artery, a distance of a tumor to a another organ, a location of a tumor within a specific subsegment of an organ, etc.. The semantic feature thus provides an additional anatomic context information, such as a position of the object of interest within the anatomy, geometric features of organs, structures or areas surrounding or adjacent to the object of interest etc. The semantic feature can especially describe a patient specific geometry in the area surrounding the object, e.g. the shape of an organ or a blood vessel.

According to an embodiment of the invention, additionally an image acquisition feature is determined and the enhanced object feature is determined from the object feature, the semantic feature, and the image acquisition feature that depends on the imaging device and/or an imaging parameter. The image acquisition feature can be provided by the imaging device or another data source, e.g. a database that provides the image.

It is however advantageous, when the image acquisition feature is determined from the image data of a third region of the image, said third region comprising a region excluding the object region. The third region can comprise a region excluding the first and/or second region and/or the first and/or second region can comprise a region excluding the third region. The third region can e.g. be a region with a given expected contrast distribution that can be used to calibrate the contrast in the image and especially in the first region of interest, e.g. an region of interest that can especially be spherical and can e.g. be placed within the aortic root or another selected anatomic structure to assess the expected contrast distribution. It is therefore suggested, to analyse the image or especially sections of the image that comprise given structures and recognize certain imaging parameters, e.g. a degree of contrast, an overall exposure, a degree of sharpness or blurring, etc. from the image data itself.

The third region of interest can be defined by a certain type, e.g. by an anatomical feature located in the third region of interest. The third region of interest can then be automatically segmented, if an automatic detection of such a feature is possible. Alternatively, it would be possible to provide a manually segmented input image or at least to provide additional information for the segmentation.

An efficient approach to achieve a large degree of independence of the object feature and the image acquisition feature or other features, e.g. the semantic feature or a clinical data feature that will be described in more detail below, is to use additional constraints, that can also be called semantic constraints, during the training of the respective algorithm used to determine these features during a machine learning. In many approaches used to train an algorithm by machine learning a cost function is minimized to optimize the performance of an algorithm. If a supervised learning is used this cost function typically depends on a difference between an output of the algorithm and a desired output provided with the training data. If an algorithm with a given parametrisation is applied to multiple sets of training data a correlation of an output of this algorithm with an output of a different algorithm that can be fixed or trained in parallel to the first algorithm and/or a correlation to an additional parameter provided with the respective training data set can be measured and a measure of this correlation can be used as an additional cost factor in the cost function. If a positive cost is assigned to a strong correlation minimizing the cost function will minimize the correlation between the output generated by the first algorithm and the output generated by the second algorithm or the provided parameter.

This approach can e.g. be used to ensure that the image acquisition features is essentially independent of the object feature by using a fixed algorithm to determine the object feature and optimizing the algorithm used to determine the image acquisition feature. On the other hand, it would also be possible to train an algorithm that is used to determine the object feature in such a way that a used object feature is approximately independent of the image acquisition feature and/or the semantic feature and/or the clinical data feature. If at the same time a strong correlation of the object feature or at least the enhanced object feature to a medically relevant output, e.g. to the likelihood of a rupture of a plaque section or a major adverse cardiac event, is maximized by the approach previously described, the additional constraints used during a machine learning can be used to craft novel biomarkers that are on the one hand approximately independent on the parameters of the image acquisition and/or more global features of the patient and on the other hand good indicators for medically relevant information.

According to an embodiment of the invention, the enhanced object feature is determined from the object feature and the semantic feature by processing image data comprising the first region of interest based on the semantic feature to obtain a processed image data set comprising the first region of interest and determining the enhanced object feature from the processed image data set. This is also referred to as use of implicit semantics in the exemplary embodiments described in this disclosure. In the simplest case the enhanced object feature can be the image data of the processed image data set or part of this image data. Alternatively, the processed image data set can be further processed to determine the enhanced object feature.

The image data in the first region of interest depends on a patient specific geometry that is described by the semantic feature, wherein the processed image data set is obtained by a transformation of the first region of interest that maps the patient specific geometry to a given standardized geometric space.

The semantic feature can provide at least one geometric feature of at least one organ, structure or area surrounding or adjacent to the object of interest. E.g. the object of interest can be a plaque within a vessel or a cancer growth. The semantic feature can define the patient specific geometry, e.g. the geometry of a coronary tree and/or a specific vessel that can e.g. comprise the plaque.

A transfer function can especially resample the image data of the first region of interest to generate the processed image data set, wherein the resampling pattern depends on the semantic feature, especially on the patient specific geometry described by the semantic feature.

A variance between data sets acquired during different acquisitions and/or on different patients that is not immediately related to the object of interest can therefore be reduced or eliminated by this remapping. This is especially advantageous, when a statistical analysis of object features should be used, e.g. in radiomics. In machine learning applications the variance of the training and input data due a respective patient specific geometry can be reduced resulting in a need for less training data and/or a more robust and/or faster conversion of the learning process.

The patient specific geometry can be used as a separate input while further processing the data. E.g. a radiomics database or an algorithm that is already trained or that is to be trained by machine learning can provide separate inputs for the processed image data and the patient specific geometry or the semantic feature.

The standardized geometric space can be defined by an anatomic atlas, e.g. a coronary atlas. Alternatively, the standardized geometric space can describe a simple geometric shape, e.g. a straight line. As an example, a curved vessel, whose geometry is e.g. defined by a centre line, can be remapped to a straight vessel or a vessel shaped according to an atlas. Alternatively, shorter sections of vessels, e.g. a respective section comprising a plaque, can be transformed.

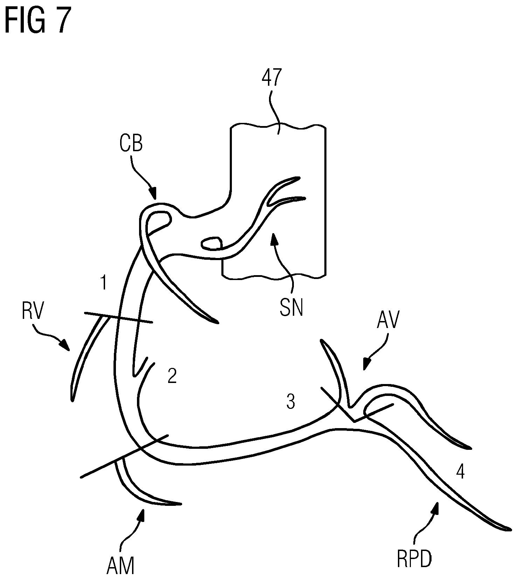

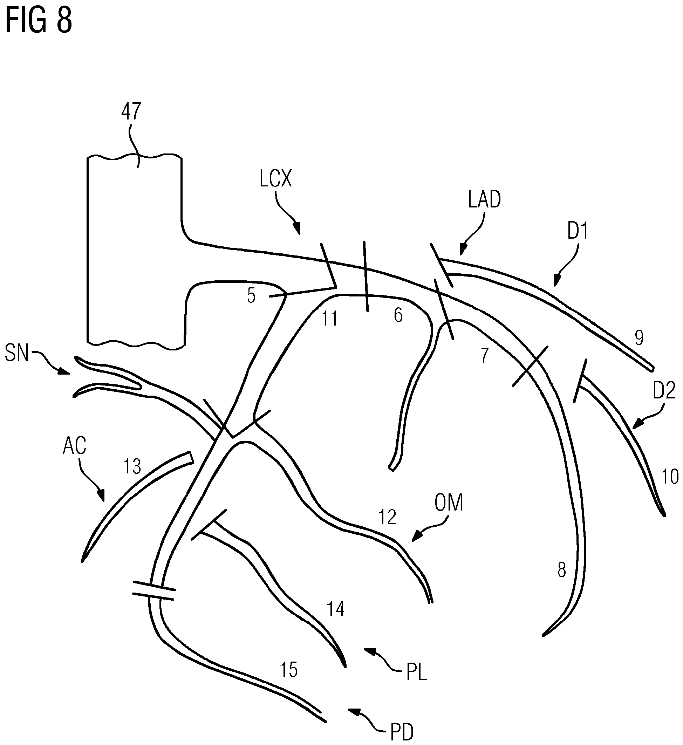

Additionally, the transformation can be used to resample and/or deform sub-image volumes in such a way that they can be arranged to canonical 2D images or 3D volumes. In an example, regions of interest associated with the three coronary main branches can be transformed in such a way, that a specific volumetric image part of the canonical image is always occupied by the straightened LAD, another by the straightened RCA, and so on. In this way holistic deep learning approaches become conceivable. Likewise, the transform and therefore the canonical image can e.g. be designed in such a way that the resulting segment-based 3D image arrangements follow an arrangement suggested by the American Heart Association shown in Fig. 7 and 8.

The processed image data set or a feature extracted from the processed image data set that is then used as an input to determine the enhanced object feature can be considered as the previously discussed intermediate feature.

According to an embodiment of the invention, the processed image data set is represented in a semantic data presentation. The semantic data representation can be considered to be a canonical image, especially a mapping of the image data to a canonical image.

A semantic data representation is defined by a semantic data model that is an abstraction that defines how the stored symbols, e.g. individual voxels in a canonical image, relate to the real world. E.g. certain voxels can be assigned to specific regions or positions in an anatomical atlas, e.g. in a model of a vessel tree or a model of an individual vessel.

The semantic data structure or canonical image can comprise data from multiple anatomic structures defined by the semantic data model, e.g. from multiple branches of a vessel tree. In a simple example individual branches of a vessel tree could be assigned to certain regions of the canonical image. Optionally the geometry of each individual vessel can be matched to a standardized vessel model or e.g. a straight line.

The enhanced object feature is determined from the processed image data set or a processed image data set, wherein the processed image data set is obtained by processing the image data of the first region of interest, wherein the processing involves a normalization of the image intensities that depends on the image acquisition feature and/or the semantic feature. Normalization is a process that changes the range of the pixel or voxel intensity values. Preferably the intensity values are scaled by a scaling factor and/or offset by an offset, wherein the scaling factor and/or the offset depend on the image acquisition feature and/or the semantic feature. Alternatively the image acquisition feature and/or the semantic feature can describe a contrast distribution in an area of interest and the normalization can be parametrized in such a way that it would match this contrast distribution to a given contrast distribution. Preferably a region of interest that corresponds to an anatomic feature, e.g. the third region of interest, can be chosen in the image, a contrast distribution in this region can be analysed, the contrast can be compared to a default contrast distribution and the result of this comparison can determine the scaling factor and/or offset.

According to an embodiment of the invention the enhanced object feature is determined from the object feature or the processed image data set or a feature determined from the processed image data set and the semantic feature based on a combination of the object feature and the semantic feature. This is also referred to as use of explicit semantics in the exemplary embodiments described in this disclosure. The combination of the object feature and the semantic feature may be achieved by a logical operation or an algorithm, e.g. an algorithm trained by machine learning, especially deep learning. The combination can e.g. be achieved by using a feature vector as an input vector that concatenates the semantic feature and the object feature or the processed image data set or the feature determined from the processed image data set.

According to an embodiment of the invention, the enhanced object feature is determined from the object feature, the semantic feature, and a clinical data feature. The clinical data feature comprises for example laboratory data, data from previous examinations, patient data (e.g. age, sex, weight, height, body-mass-index, previous diagnosis data etc.), or epidemiological data.

According to an embodiment of the invention, the step of enhanced object feature determination from the object feature and the semantic feature is performed using an algorithm trained by machine learning, especially deep learning. The algorithm can be e.g. trained by supervised learning. A multitude of training datasets can be used, each comprising an input vector and a desired output. The training can be achieved by minimizing a cost function that can depend on the difference between the actual output and the desired output. A multitude of methods for training algorithms by machine learning are known. Those approaches will therefore not be discussed in detail. As a simple example an artificial neural network can be used. Training can then e.g. be achieved by the well-known approach of backpropagation of error. Using machine learning provides the advantage, that it is not necessary to manually parametrize the algorithm. Instead it is sufficient to provide good training data, that can e.g. be provided by manually classifying input vectors or data that was used to generate the input vectors by medical professionals.

An input vector for the algorithm can be directly formed by concatenating the semantic feature and the object feature. Optionally the image acquisition feature and/or the clinical data feature can be used as part of the input vector. It can however be advantageous to pre-process the object feature, e.g. by generating the processed image dataset or by determining an intermediate feature from the processed image dataset as discussed above and then using these intermediate features instead of the object feature or in addition to the object feature.

According to an embodiment of the invention, the semantic feature comprises an object positional information indicating an object position relative to an anatomic structure. This can e.g. improve the classification of the object or the determination of properties or an expected behaviour of an object. It might e.g. be more or less likely for a plaque to rupture depending on its position with respect to the next upstream and/or downstream branch of the vessel tree.

In the context of our invention it is possible to use global image semantics to identify appropriate local and global data representations from CCTA data (single- or dual-/multi-energy) to train machine learning-based, especially deep convolutional, neural networks, etc. Classification or regression systems that may immediately predict the likelihood of future major adverse cardiac events or rate the presence of other pathologic confounders suggesting such events in the future, e.g., pathogenic plaque morphologies like the napkin ring sign, etc. can be trained. Alternatively, or additionally these data representations can be mined in cohort studies for new biomarkers that correlate with such events or the presence of pathologic confounders.

The invention also concerns a method for training an algorithm by machine learning, wherein multiple training datasets are used to train the algorithm, wherein each training dataset comprises an image, wherein a respective enhanced object feature is determined from the respective image by the method according to the resent invention and used as an input or processed further to generate an input to the algorithm during the machine learning. The general approach for implementing a supervised learning was already discussed above. This approach is now modified by not directly providing the complete input vector as part of a respective training dataset. Instead at least part of the input vector is provided by the enhanced object feature.

This approach is especially relevant, when a property or an effect of the object of interest should be determined by the algorithm that is expected to be independent from the semantic information. The pre-processing by the inventive method can especially be used to reduce a variance in the input datasets due to changes in the semantic information. If the image acquisition feature and/or clinical data feature are also taken into account, an influence of these features might also be eliminated.

In general the variance between training data sets that is not caused by the object itself can therefore be reduced or eliminated and a robust learning can therefore be achieved with smaller training datasets. This is especially advantageous, when data about rare diseases should be learned, because there is typically only a limited amount of training data available in this case.

In a simple case the object information can be the image data in the first region of interest or the object region and the enhanced object information can be the processed image data set. The processed image data set can especially be generated by mapping the patient specific geometry to a given standardized geometric space. An influence of the patient specific geometry can therefore be largely eliminated.

The described approach can also be used to generate additional training data. It is e.g. possible to map the first area of interest to a specified, especially linear, geometry, than apply certain modifications to the data, e.g. a stepwise rotation around an axis of symmetry of a straightened vessel, and then perform a transform back to the patient specific geometry. This approach might be useful when a pre-processing of data is not intended in the final application of the algorithm. It does however create additional relevant training data in many cases.

As previously described an explicit decorrelation of different features can be performed while the machine learning is performed.

The invention also concerns an algorithm trained by this method and a computer-readable storage medium containing information representing this algorithm, e.g. a program or other instructions implementing the trained algorithm or parameters determined during the training that parametrize a given algorithm comprising multiple free parameters, e.g. a neural network.

The invention further relates to a system comprising a processing unit, especially a processing unit of a medical imaging device, configured to perform the inventive method.

The system may be implemented on a computer, a workstation, in a network of computers or the like. It can be provided as part of an imaging scanner and can be integrated into the scanners control system. It can also be implemented in a cloud-based solution.

The invention further relates to a computer program that can be directly loaded into a memory unit of a processing unit, especially a processing unit of a medical imaging device, the computer program comprising instructions for performing the steps of the inventive method when the program is executed on the processing unit.

The invention further relates to a computer-readable storage medium containing electronically readable instructions comprising the computer program.

Additionally, radiomic cohort studies can be successively augmented by processing additional patient data series with the methods as described above.

Specific embodiments of the invention will be described in detail herein below with reference to the figures wherein the figures show schematically:
- Figs. 1 and 2: Representations of an atherosclerotic plaque as an exemplary object to be analyzed by the method according to the present invention,
- Figs. 3 and 4: schematic representations of exemplary embodiments of a method according to the present invention,
- Fig. 5: a flow chart of an exemplary embodiment of the method according to the present invention,
- Fig. 6: an exemplary embodiment of the system according to the present invention, and
- Figs. 7 and 8: coronary segments according to the system proposed by the American Heart Association.

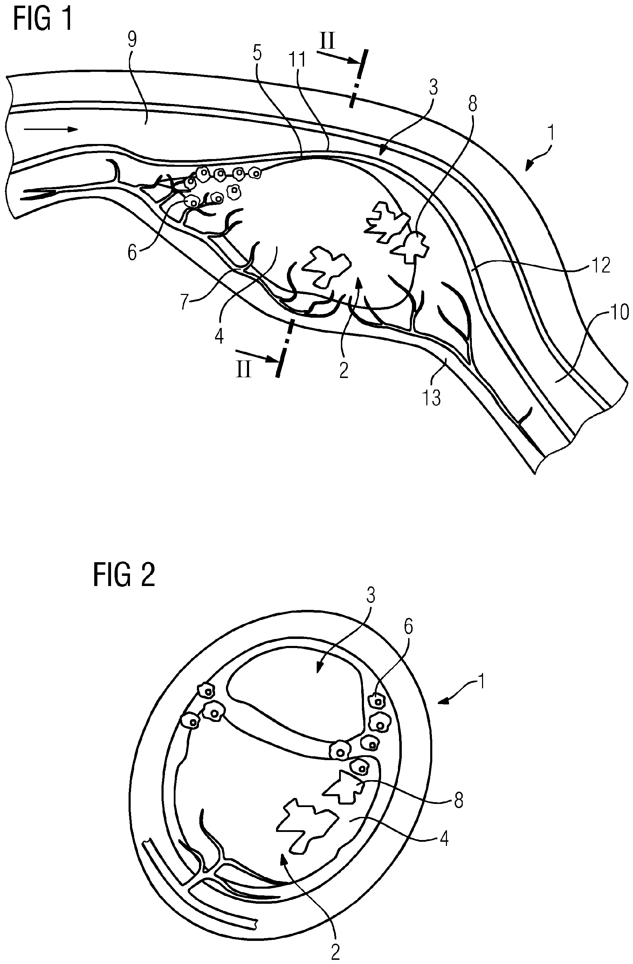

Figs. 1 and 2 show a representation of an atherosclerotic plaque as an exemplary object that can be analyzed by the methods discussed below.

The plaque 2 is attached to a vessel wall 13 of a vessel 1 therefore limiting the width of a lumen 3. This is clearly seen in fig. 1 and in fig. 2 that show a cut along the line II-II in fig. 1. The presence of such a plaque 2 causes a low fractional flow reserve 10 on the downstream side of the plaque 2 when a normal fractional flow reserve 9 is present on the upstream side of the plaque 2. In the example in fig. 1 and 2 a plaque 2 is shown which is vulnerable to rupture and therefore causes a rather large risk for major adverse cardiac events. Some exemplary qualities of such a plaque 2 are schematically shown in fig. 1 and 2.

The plaque 2 comprises a large lipid-rich necrotic core 4 with a thin fibrous cap 5. Vasa vasorum proliferation 7 extends into the core 4 and the core 4 comprises spotty calcium 8. In the downstream plaque region the endothelial sheer stress 12 is low and oscillatory and therefore promotes plaque growth. In the upstream region low endothelial sheer stress regions are more inflamed which causes the presence of macrophages 6. A high endothelial sheer stress 11 at the most stenotic part can trigger a plague rupture.

Several of the mentioned features influence the absorption of x-ray radiation in the area of the plaque 2 and therefore images acquired by x-ray imaging, especially three-dimensional data sets acquired during coronary computer tomography angiography. It is therefore known to manually or automatically segment regions of the three-dimensional image comprising a plaque 2 and extracting local features, that are also called object features in the context of the present invention, from such regions. Such features can e.g. be texture features of the object, features recognized after a Fourier or wavelet transform of the respective region, features determined by deep learning, etc. These local features can be considered biomarkers, when they are strongly correlated to a relevant property, e.g. the likelihood of a rupture of the plaque 2. By associating these biomarkers with diagnostically relevant information, e.g. by using statistics or a machine learning based approach, diagnostically relevant information, e.g. the likelihood of a plaque rupture or a potentially life-threatening major adverse cardiac event, can be directly determined from these local features.

It was recognized that good predictions require a very large amount of input data and are not sufficiently robust in many cases. It was recognized, that these drawbacks can be eliminated or at least noticeably reduced by not only using local features, also called object features in the present invention, but also taking more global information, also called semantic features, that can also be extracted from the image into account. Several examples for this approach will be given below.

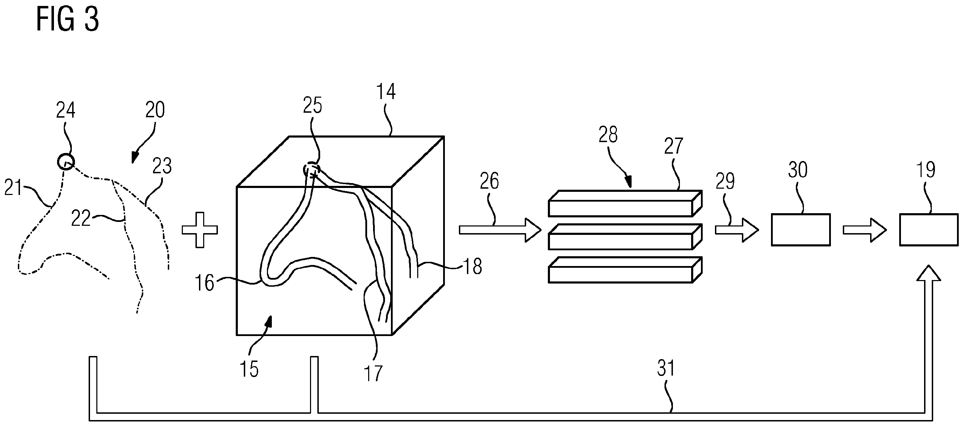

Fig. 3 gives a general overview of some of the ideas that can be used to generate an enhanced object feature 19 from an image 14 comprising three-dimensional image content, in the example a contrasted lumen 15. The image 14 comprises three objects 16, 17, 18, namely three branches of the vessel tree formed by the lumen 15.

In a first step semantic features 20 are determined from the image data of the image 14, that describe an anatomic context information. The semantic features 20 on the one hand describe a patient specific geometry, namely the main coronary center lines 21, 22, 23 of the objects 16, 17, 18, that can be automatically or manually extracted from the image 14. Additionally, the semantic feature 20 describes the position 24 of the aortic root. This can be used to define a region of interest 25 that can be used for contrast corrections. This will be described later.

The semantic information 20 is used in two ways in the example according to fig. 3. First, there is an implicit use of a global three-dimensional semantic feature 20 to generate an appropriate semantic data representation. In this step 26 a patient specific geometry, namely the shape of the vessels or objects 16, 17, 18 that is described by the semantic features 20, namely the center lines 21, 22, 23, is mapped to a standardized geometric space 27, in this case a linear representation, to generate a processed dataset 28. This eliminates or at least reduces the influence of the patient specific geometry and therefore of a global feature onto the local features that will be determined from the processed image dataset 28.

This transformation can also be used to additionally or alternatively eliminate the influence of the used imaging device and/or imaging parameters. One of the most relevant influences of using different imaging devices and/or imaging parameters can be the contrast distribution in the image 14. This influence can be reduced or eliminated by using the region of interest 25 at a known anatomic position with an expected contrast distribution to normalize the processed image dataset 28. The contrast distribution in the region of interest 25 can be analyzed and compared to a reference contrast distribution. This can e.g. be used to determine an offset and/or a slope that can be used to map the voxel values in the image 14 to the voxel values in the processed image dataset 28.

The processing could be finished in this step, since an object feature, e.g. a feature vector describing the voxel values in a respective region of interest for the respective object, and the semantic features 20 are used to generate an output that could be considered to be an enhanced object feature. Such a purely implicit use of the semantic features 20 can e.g. be advantageous when the processed image dataset 28 should be used for training an algorithm by machine learning. This could e.g. be advantageous when an algorithm should be trained to be sensitive to local features, e.g. object information, but should not be sensitive to the patient specific geometry. Input data for this algorithm can later be preprocessed in the same way to mostly eliminate the patient specific geometry from input data. This can especially allow for a training of the algorithm with a smaller number of training datasets, since a variance due to different patient specific geometries and/or image acquisition conditions can be mostly eliminated.

In a next step 29 a feature computation is performed, e.g. by applying an algorithm trained by machine learning to the processed image dataset 28. This could e.g. be an algorithm that was trained to detect and/or classify and/or determine properties of plaque detected within the vessel. Since the processed image dataset 28 comprises the full vessels, it is e.g. also possible to determine a relative position of the plaque with respect to the respective vessel. The extracted intermediate features 30 can e.g. be represented as a feature vector v=(v₁, v₂, ..., vₙ) . As previously discussed with respect to the processed image dataset 28, the intermediate feature 30 could already be considered to be an enhanced object feature, since it is determined in dependence of the semantic features 20.

In the example an additional step 31 is used that makes explicit use of the global 3D semantic features 20, e.g. of the main curvature, an explicit information about the affected branch, a main intensity in the calibration region 25, etc. to determine the enhanced object feature 19. Therefore, the feature 30 could also be considered to be an object feature that is processed in conjunction with the semantic features 20 to generate the enhanced object feature 19.

In the simplest case the enhanced object feature 19 could be generated by concatenating the feature vector v for the feature 30 with the feature vector v' comprising the semantic features 20 or features generated from the semantic features 20 to form a combined feature vector **v***=(**v**, **v'**). It is however advantageous to perform further processing, e.g. by using the combined feature vector as an input to a further algorithm, that can e.g. be previously trained by machine learning and that can e.g. be used to directly generate diagnostically relevant information, e.g. a probability of a rupture of a plaque or the probability of a major adverse cardiac event.

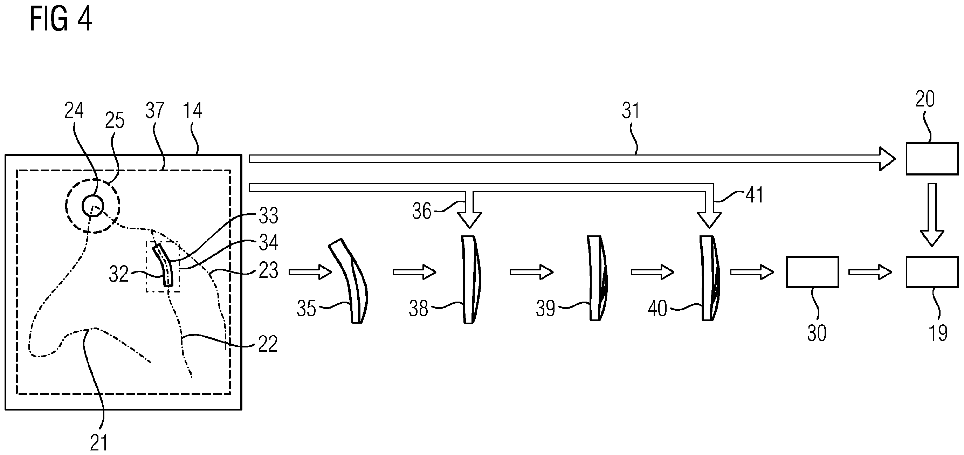

Fig. 4 describes a slightly different approach to determine an enhanced object feature 19. In fig. 4 the image 14 is shown in a highly abstracted manner wherein the actual image content is only schematically shown in an object region 33 comprising the object 32 of interest, namely a plaque section. The object region 33 can therefore also be considered to be a plaque area. In the other regions of the image 14 only the extracted semantic features 20 are shown. The semantic features are extracted from a region 37 of interest that comprises regions outside the object region 33. The extraction of semantic features was already discussed with respect to fig. 3. The object region 33 can automatically be detected or manually segmented from the image 14.

In a first step an object feature 35 is determined from the image data of a region 34 of interest that comprises the object region 33. As schematically shown in fig. 4 the object feature is a feature vector comprising the voxel values of the individual voxels in the object region 33 or the region 34 of interest.

The object feature is then processed to perform a three-dimensional geometric standardization. As shown by the arrow 36 a semantic feature, namely the shape of the center line 22 in the object region 33, is used to generate a first processed image dataset 38, in which the patient specific geometry shown in the original image 14 is mapped to a standardized geometric space, namely a straight line. This can be achieved by resampling the image data in the object region 33 or the region of interest 34 that forms the object feature 35. The pattern of the resampling can be easily determined from the known shape of the center line 22. A masking is then performed to generate a three-dimensional masked image 39. Masking is used to ensure that mainly features of the vessel wall are shown. Approaches for masking vessel images to focus on the vessel wall and plaque are well-known in the prior art and will not be discussed in detail.

To eliminate or at least to reduce the influence of an imaging device and/or imaging parameters an image acquisition feature is determined from the region 25 in which a certain contrast distribution is expected. By comparing the actual contrast distribution in the region 25 with an expected contrast distribution voxel values can be remapped as already explained with reference to fig. 3 to generate a corrected processed image dataset 40. As shown by the arrow 41 semantic features, namely the position 24 of the aortic root, is implicitly used in this step.

This dataset 40 can then be used to determine the intermediate feature 30 that can be combined with the semantic features 20 to form an enhanced object feature 19 as previously discussed with reference to fig. 3. Since only a short section of the respective vessel is used to extract the object feature 35 in the method described with respect to fig. 4 this combination of the intermediate feature 30 with the semantic feature 20 can also comprise the addition of information concerning the specific position of the object 32 of interest and therefore the plaque.

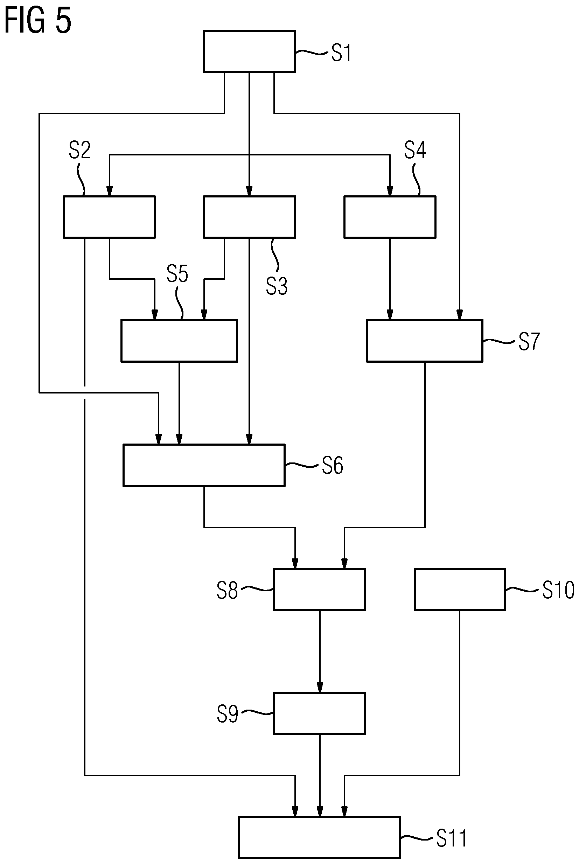

Fig. 5 shows a flowchart of a method for determining at least one enhanced object feature of an object of interest that is at least partially depicted by an image in a region of an anatomy of a patient. In step S1 such an image is provided. The image is preferably a three-dimensional image, especially a computer tomography image. The object can e.g. be a plaque in a vessel of the patient. Image data usable to extract an enhanced object feature, e.g. a likelihood that the plaque ruptures or a likelihood of a potentially life-threatening major adverse cardiac event, can e.g. be determined when the image is acquired by coronary computer tomography angiography. It is however not necessary to use a freshly acquired image. Alternatively, e.g. an image from a database can be used. This can e.g. be advantageous when the method is used to determine enhanced object features that are used to train a further algorithm, e.g. by machine learning.

In the steps S2, S3 and S4 respective semantic features that comprise the respective anatomic context information are extracted. The steps S2, S3 and S4 can be performed in parallel or independent from each other. It is however also possible, that one of these steps depends on the result of another one of these steps. In step S2 a patient specific geometry, that in this example concerns the structure of a coronary artery, is extracted. The semantic feature extracted in step S2 can especially describe the shape of the individual vessels, especially the shape of the center lines of the vessels. The determination can be completely automatic or a manual input of a user can be required. Several approaches for determining center lines from tomography images are known in the prior art. These approaches will not be discussed in detail.

In step S3 the position of an object of interest, e.g. a plaque, is determined. The object of interest can be pre-segmented in the image or it can be automatically detected. Several approaches for automatically detecting and segmenting plaque in tomography images are known in the prior art and will not be discussed in detail.

In step S4 the position of the aortic root is detected. This can e.g. be easily achieved when using the center lines of the individual vessels determined in step S2. It can however also be advantageous to directly recognize the aortic root in the image. As will be discussed in more detail later the image in the area of the aortic root is used for a contrast correction. In principle other areas or anatomic structures of the patient could also be detected that could be used as reference regions. It is also be possible to use multiple regions for a contrast reference.

Using the shape of the center lines determined in step S2 and the position of the object of interest determined in step S3 the shape of the vessel that comprises the object of interest in the area of the object of interest is determined and a transformation that resamples the patient specific geometry defined by this shape to a standardized geometric space is determined in step S5. Preferably, the transform can be determined in such a way that the curved vessel section is essentially straightened as already discussed with respect to figs. 3 and 4.

In step S6 a processed image dataset is generated by first selecting a region of interest in the image based on the position of the object of interest determined in step S3 and then using the transform determined in step S5 to resample this region of interest or an object region that depicts this object in the region of interest.

The processed image dataset determined in step S6 is largely independent from the patient specific geometry. It does however still strongly depend on the imaging device and the imaging parameters used to acquire the image. To reduce this dependency, information about the image acquisition is first extracted from the image. A region of interest is defined in the area of the aortic root or some other well-defined anatomic feature determined in step S7 and the contrast distribution in this region of interest is determined and compared to a given expected contrast distribution. From this comparison an offset and a slope for the voxel values of the voxels is determined in such a way that the contrast distribution determined from the image approximately matches the given contrast distribution. In step S8 this offset and slope are then applied to the voxel values of the transformed image dataset determined in step S6 to determine a further transformed image dataset.

In step S9 an algorithm that can e.g. be previously trained by machine learning, can be applied to this dataset to determine a feature vector for the object of interest. In step S9 this feature vector can then be merged, e.g. with at least part of the semantic features determined in the steps S2 and/or S3 and/or S4 and/or with clinical data features provided in step S10 that can e.g. be provided by a patient database that provides data about previous examinations, patient data, etc. A feature vector comprising all the mentioned features can then be processed by an algorithm, especially an algorithm trained by machine learning, to determine the enhanced object feature in step S11. The enhanced object feature can especially be of diagnostic relevance and e.g. provide information about the likelihood of a rupturing of the plaque and/or of a major adverse cardiac event.

It would also be possible to skip step S9 and directly use the processed image dataset determined in step S8 as an input for step S11. While this might in some cases be advantageous, it requires an algorithm with more inputs and e.g. a more complex neural network that needs to be trained.

As previously mentioned an algorithm trained by a machine learning can be used in step S9 to determine a feature or a feature vector from the processed image data set determined in step S8. The preprocessing of the image data to determine the processed image data set removes or at least reduces a dependency of the processed image data set on the imaging conditions and on the patient specific large scale geometry surrounding the local feature depicted by the processed image data set.

To train such a network, supervised learning can be used. Instead of a single image multiple sets of training data will be processed. Each of these sets of training data can additionally to the image comprise a desired feature or feature vector that should be determined in step S9 for the respective image. The steps S1 to S8 can then be performed for each individual training data set and a difference between the feature vector determined in step S9 and the feature vector provided with the respective training data set can then be minimized by modifying parameters of the algorithm used in step S9. Except for the additional preprocessing this is equivalent to a normal supervised learning procedure. Such machine learning procedures are well known in the prior art and will not be discussed in detail.

The discussed machine learning can be further improved when an additional condition is applied during the learning. The features determined in step S9 should show no or only a weak correlation to the semantic features determined in step S2, S3 and/or S4 and/or to the acquisition feature determined in step S7. This can be achieved by applying the algorithm used in step S8 for a sub set of the training data and then determining a respective measure for the mentioned correlations. This measure for the respective correlation can be used as an additional factor in a cost function used to optimize the algorithm used in step S8.

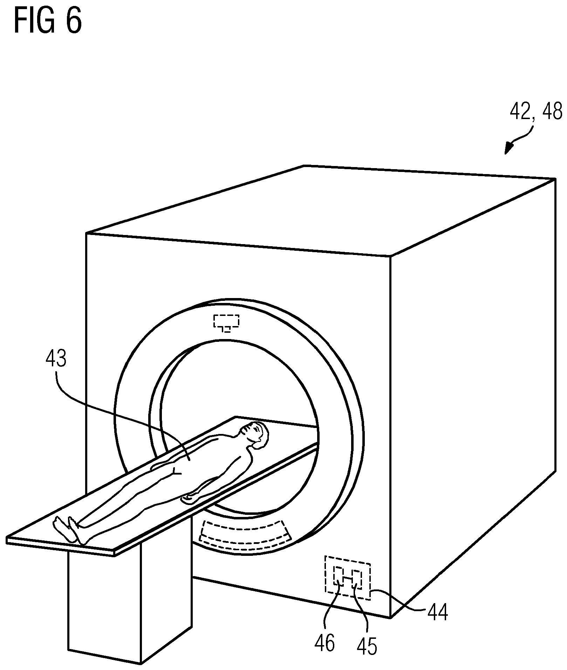

Fig. 6 shows an example of a system 42, in this example a medical imaging device 48, especially a computer tomography scanner, that is useable to perform coronary computed tomography angiography on a patient 43. The system 42 comprises a processing unit 44 configured to perform at least one embodiment of the methods discussed above. The processing unit 44 comprises a processor 45 and a memory unit 46 that can be loaded with a computer program comprising instructions for performing the discussed method when the program is executed on the processing unit 44.

The processing unit 44 could also be a separate unit from the medical imaging device 48. It could e.g. be implemented on a separate computer, on a work station in a network of computers or in a cloud-based solution. The processor 45 can be any programmable device, e.g. a CPU, an FPGA, a micro controller, etc. The individual steps of the discussed method can all be performed by the same processor 45, distributed among identical processors or performed on different processors.

Fig. 7 shows a segmentation of the right coronary artery and Fig. 8 shows a segmentation of the left anterior descending coronary artery proposed by the American Heart Association.

This proposed segmentation can be used as a canonical image or a kind of atlas to provide a standardized geometric space to which the image data of different vessels can be mapped.

Each image shows the aorta 47 and the branching vessels that are labeled as follows:
AC: atrial circumflex branch; AM: acute marginal branch; AV: atrioventricular node; CB: conus branch; D1: first diagonal branch; D2: second diagonal branch; LAD: left anterior descending coronary artery; LCA: left coronary artery; LCX: left circumflex coronary artery; OM: obtuse marginal branch;
PD: posterior descending branch; PL: posterolateral branch;
RCA: right coronary artery; RPD: right posterior descending branch; RV: right ventricle; SN: sinus node.

In the present invention a focus lies in the presentation of concepts that allow using global characteristics or semantic features to convert the data of interest in canonical manners to alternative data representations (e.g. by non-linear reformatting and resampling, HU values standardization relative to calibrations regions, etc.) that may be better suited to identify biomarkers from the presented data to provide enhanced object information or enhanced object features and e.g. for developing cardiac decision-support systems, such as decision-support systems. Especially in deep learning a suitable data representation can improve the results of a classification or regression task. This can be referred to as "implicit use of global image semantics", wherein the enhanced object feature is determined from the object feature and the semantic feature by processing image data based on the semantic feature to obtain a processed image data set and determining the enhanced object feature from the processed image data set, i.e. the data representation is guided by global semantic image characteristics. We call this qualified data representation a "semantic data representation".

The general scheme of our invention is depicted in Figure 3.

Starting e.g. from the volumetric image data and explicit geometric information like vessel centerlines or calibration ROIs we suggest making use of the given global semantics for appropriate radiomics biomarker identification in two ways:
1. Explicit use of global semantics - classical feature extraction: global features/semantic features like the mean curvature of the vessels complement the object feature of the object (e.g. texture features of the object, Fourier/wavelet features, deep learning features, ...), e.g., computed on the region of interest containing the object region or other image sub-volumes of interest.
2. Implicit use of global semantics/semantic features to achieve guidance for semantic data representation: global information/semantic features are used to change local and global data representation in an effective way for subsequent processing and feature extraction steps. This is of particular interest in machine learning set-ups where stripped and standardized data representations containing essential information only are likely to speed-up model generation as typically less training data is required to reach convergence. The main application here is deep learning that automatically extracts meaningful information regarding a diagnostic question from the represented image data. A fast convergence is especially important for rare diseases where not enough data can be collected, a problem often occurring in medicine.

As the usual first step of the invention a potentially fully-automated approach for step-wise semantic enrichment of the data can be used. In the context of coronary plaque assessment from CCTA data this may involve: i) coronary tree extraction in terms of vessel centerlines, ii) plaque sections definition/detection, e.g., in terms of vessel wall masks, and (iii) definition/detection of calibration ROI(s) (e.g., a spherical ROI within the aortic root to assess the vessels' expected contrast concentration).

The gained semantics, and especially global aspects therein, are now used to construct a diagnostic system in several manners e.g. to complement or to enrich object features which are local features of the object of interest (such as size, globularity, homogeneity, texture, shape, HU statistics, etc.) from the region of the object of interest (e.g. an individual plaque section, tumor region, or the like):
(1.) A Global information or a semantic feature like for instance the distance to the aortic root along the connecting vessel can be concatenated with an object feature, e.g. the classical local feature vectors originating from the region of the object of interest, e.g. the plaque sections, themselves. The results of subsequent data mining for biomarkers and statistical analysis will consequently be more exhaustive. (see previous example and right part of Figure 4; "explicit use of global semantics") .
(2.) It is envisioned to determine the enhanced object feature from the object feature and the semantic feature by processing image data comprising the first region of interest based on the semantic feature to obtain a processed image data set comprising the first region of interest and determining the enhanced object feature from the processed image data set. This represents an approach aiming at mapping a patient specific geometry (such as a coronary tree) to a standardized geometric space (such as a coronary atlas) ("3D geometric standardization"; see Figure 3 and Figure 3). Associated resampling immediately enriches observed plaque morphology by global aspects relative to the whole coronary tree. Higher level knowledge about the depicted scenery like the mentioned topology of the coronary tree is therefore used to geometrically standardize the data before analyzing the data (e.g. by deep learning) approaching an "atlas-like" (cf. "Talairach space" for brain structures) representation ("implicit use of global semantics"). The global information can be used as a prior for data representation that can optimize itself after first data analysis. The global semantics are used in three procedures (i, ii, iii):
   i. Semantic spatial global and local data representation:
      Volumetric image data of individual vessels can be "unfolded" via appropriate resampling schemes relying on the course of the associated centerline. This yields elongated 3D sub-images depicting a straightened version of the vessel. The idea is schematically depicted in Figure 3. The same can be done with shorter individual plaque sections as depicted in the central part of Figure 4. Both representations have the advantage to lower the morphological variance that is not of interest when trying to process these whole vessel sub-image volumes, e.g., in machine learning or especially deep learning set-ups. This becomes apparent when considering the fact that plaque vulnerability is said to be determined by plaque composition rather than by vessel curvature. In addition, axes-aligned bounding boxes covering the non-straightened vessel sections may contain too much non-problem-related image content to be practical for deep learning approaches. Particularly, in the case of deep learning where large amounts of training data are needed the straightened representation also allows data augmentation: step-wise rotation of the vessel sections around the centerline and associated repeated resampling will, for instance, yield categorical identical samples (e.g., low risk vs. high risk vessel sections) with different starting representations for feature computation. Data augmentation is of interest whenever the data representation is chosen in a potentially practical and explanatory way but invariance under geometric transformations of the above kind cannot be guaranteed.
   ii. Semantic global and local data representation of CT value distribution: Further calibration regions, not necessarily in immediate proximity to the plaque section, like a spherical ROI in the aortic root (see upper-left part of Figure 4) may contain covariates, e.g., for standardizing the sub-volumes' HU range before computation of other plaque features. The intensity distribution is adapted to this information. It has already been shown, that such a transformation of data representation can significantly improve deep learning results. More sophisticated feature space standardization techniques based on a potential multitude of calibration regions are possible. ("implicit use of global semantics"). The identified covariates for technical information or contrast bolus injection will be used in (3.).
(3.) In a final step we propose to integrate the results of explicit and implicit use in a machine learning system especially deep learning. The deep learning system uses the aligned semantic local and global data representations to automatically identify diagnostic relevant image content. The global feature and the technical covariates of the calibration regions are integrated in the inner layers of the deep learning system, thus the system concentrates on the additional information that is not already explained by technical characteristics or can be identified with simple global features. In this step the possibility to integrate further information such as laboratory parameters or epidemiologic data is given. Also information which is lost in the transformation step to a semantic data representation should be integrated (e.g. curvature of a vessel tree, when the semantic global data representation is a straightened coronary tree).

Figure 4 depicts a conceivable processing pipeline for a lesion-centered perspective: the course of the vessel centerline in the plaque sections is used to geometrically normalize ("straighten") the image areas, where the local plaque features are about to be extracted. Here the focus lies on the masked vessel wall. The image intensities may have been subject to normalization, e.g., with respect to the intensity statistics in the contrasted aortic root ("implicit use of global semantics"). Additionally, global features, of any kind and at any level of hierarchical semantic abstraction, can be considered in subsequent data mining steps via vector concatenations.

In the following we summarize the new advantages of our newly invented approach for risk stratification and biomarker discovery for atherosclerotic plaque from CCTA: 1. We suggest integration of disease characteristics to be found in the imaging data at different levels of geometric abstraction enabling a holistic assessment of the underlying disease. This is achieved by making explicit and implicit use of global image semantics for image analysis in addition to local features from the potentially reformatted target ROIs. In the context of the whole coronary tree and atherosclerotic risk stratification doing so has the potential to yield an Agatston score equivalent on contrasted scans, which would call the actual need for a preceding non-contrast scan (radiation dose!) into question. 2. Specifically, we suggest converting the imaging data to more appropriate semantic data representations based on global semantics before radiomics and especially deep learning analysis. With this implicit use of global semantics for data reformatting, new machine learning and data mining technologies especially deep learning that are sensitive to extensive non-problem-related image content can be successfully applied. 3. Following the same line of argumentation ("implicit use of global semantics"), additional non-geometric global characteristics from calibration regions are used to further stabilize the chosen data representations or even the finally extracted features. The goal is to reproducibly and comparably analyze a patient's coronary status in a standardized representation for precision medicine, i.e., subtle changes in the disease manifestation in this representation or the identified feature space, i.e. differences in radiomic feature expression, may have different implications on diagnosis, prognosis, and therapy. 4. It is expected that our standardized semantic data representation enables decision support systems that need less data which is especially of interest for rare diseases. This is caused by a reduction of variance that is not of interest and the automated engineering of diagnostic relevant features by means of deep learning that are also orthogonal to technical characteristics. 5. Besides, global features can be explicitly used to complement the extracted local features. These global features have been neglected in the literature.

## Claims

1. Method for determining at least one enhanced object feature (19) of an object (16, 17, 18, 32) of interest that is at least partially depicted by an image (14) in a region of an anatomy of a patient (43), said image (14) having been provided from an imaging device (48),
wherein a respective object feature (35) for each object (16, 17, 18, 32) and at least one semantic feature (20) are determined from the image data of the image (14),
wherein said object feature (35) depends on information comprised in image data of a first region (34) of interest, said first region (34) of interest containing the object region (33), and the semantic feature (20) depends on information comprised in image data of a second region (37), said second region (37) of interest comprising a region excluding the object region (33), and
wherein said semantic feature (20) comprises an anatomic context information, wherein the enhanced object feature (19) is determined from the object feature (35) and the semantic feature (20),
wherein the enhanced object feature (19) is determined from the object feature (35) and the semantic feature (20) by processing image data of the first region (34) of interest based on the semantic feature (20) to obtain a processed image data set (28, 38, 40) comprising the first region (34) of interest and determining the enhanced object feature (19) from the processed image data set (28, 38, 40),
wherein the image data in the first region (34) of interest depends on a patient specific geometry that is described by the semantic feature (20), wherein the processed image data set (28, 38, 40) is obtained by a transformation of the first region (34) of interest that maps the patient specific geometry to a given standardized geometric space (27).

2. The method according to claim 1, wherein additionally an image acquisition feature is determined and the enhanced object feature (19) is determined from the object feature (35), the semantic feature (20), and the image acquisition feature that depends on the imaging device (48) and/or an imaging parameter.

3. Method according to claim 2, wherein the image acquisition feature is determined from the image data of a third region (25) of the image (14), said third region (25) comprising a region excluding the object region (33) .

4. The method according to any of the above claims, wherein the processed image data set (28, 38, 40) is represented in a semantic data presentation.

5. The method according to any of the above claims, wherein the enhanced object feature (19) is determined from the processed image data set (28, 38, 40) or a processed image data set (28, 38, 40), wherein the processed image data set (28, 38, 40) is obtained by processing the image data of the first region (34) of interest, wherein the processing involves a normalization of the image intensities that depends on the image acquisition feature and/or the semantic feature (20).

6. The method according to any of the above claims, wherein the enhanced object feature (19) is determined from the object feature (35) and the semantic feature (20) based on a combination of the object feature (35) or the processed image data set (28, 38, 40) or a feature (30) determined from the processed image data set (28, 38, 40) and the semantic feature (20).

7. The method according to any of the above claims, wherein the enhanced object feature (19) is determined from the object feature (35), the semantic feature (20), and a clinical data feature.

8. The method according to any of the above claims, wherein the step of enhanced object feature (19) determination from the object feature (35) and the semantic feature (20) is performed using an algorithm trained by machine learning, especially deep learning.

9. The method according to any of the above claims, wherein the semantic feature (20) comprises an object positional information indicating an object position relative to an anatomic structure.

10. Method for training an algorithm by machine learning, wherein multiple training datasets are used to train the algorithm, wherein each training dataset comprises an image (14), wherein a respective enhanced object feature (19) is determined from the respective image (14) by the method according to one of the preceding claims and used as an input or processed further to generate an input to the algorithm during the machine learning.

11. System comprising a processing unit (44), especially a processing unit (44) of a medical imaging device (48), configured to perform the method of one of the preceding claims.

12. Computer program that can be directly loaded into a memory unit (46) of a processing unit (44), especially a processing unit (44) of a medical imaging device (48), the computer program comprising instructions for performing the steps of the method of one of the claims 1 to 10 when the program is executed on the processing unit (44).

13. Computer-readable storage medium containing electronically readable instructions comprising the computer program according to claim 12.

## Patentansprüche

1. Verfahren zur Bestimmung von mindestens einem erweiterten Objektmerkmal (19) eines Objekts (16, 17, 18, 32) von Interesse, das mindestens teilweise durch ein Bild (14) in einer Region einer Anatomie eines Patienten (43) abgebildet wird, wobei das Bild (14) von einer Bildgebungsvorrichtung (48) bereitgestellt worden ist,
wobei ein jeweiliges Objektmerkmal (35) für jedes Objekt (16, 17, 18, 32) und mindestens ein semantisches Merkmal (20) aus den Bilddaten des Bildes (14) bestimmt werden,
wobei das Objektmerkmal (35) von Informationen abhängt, die in Bilddaten einer ersten Region (34) von Interesse enthalten sind, wobei die erste Region (34) von Interesse die Objektregion (33) enthält, und das semantische Merkmal (20) von Informationen abhängt, die in Bilddaten einer zweiten Region (37) enthalten sind, wobei die zweite Region (37) von Interesse eine Region umfasst, welche die Objektregion (33) ausschließt, und wobei das semantische Merkmal (20) eine anatomische Kontextinformation umfasst,
wobei das erweiterte Objektmerkmal (19) aus dem Objektmerkmal (35) und dem semantischen Merkmal (20) bestimmt wird,
wobei das erweiterte Objektmerkmal (19) aus dem Objektmerkmal (35) und dem semantischen Merkmal (20) bestimmt wird, indem Bilddaten der ersten Region (34) von Interesse basierend auf dem semantischen Merkmal (20) verarbeitet werden, um ein verarbeitetes Bilddatenset (28, 38, 40) zu erhalten, das die erste Region (34) von Interesse umfasst, und das erweiterte Objektmerkmal (19) aus dem verarbeiteten Bilddatenset (28, 38, 40) bestimmt wird,
wobei die Bilddaten in der ersten Region (34) von Interesse von einer patientenspezifischen Geometrie abhängen, die durch das semantische Merkmal (20) beschrieben wird,
wobei das verarbeitete Bilddatenset (28, 38, 40) durch eine Transformation der ersten Region (34) von Interesse erhalten wird, welche die patientenspezifische Geometrie auf einen gegebenen standardisierten geometrischen Raum (27) mappt.

2. Verfahren nach Anspruch 1, wobei zusätzlich ein Bilderfassungsmerkmal bestimmt wird, und das erweiterte Objektmerkmal (19) aus dem Objektmerkmal (35), dem semantischen Merkmal (20) und dem Bilderfassungsmerkmal bestimmt wird, das von der Bildgebungsvorrichtung (48) und/oder einem Bildgebungsparameter abhängt.

3. Verfahren nach Anspruch 2, wobei das Bilderfassungsmerkmal aus den Bilddaten einer dritten Region (25) des Bildes (14) bestimmt wird, wobei die dritte Region (25) eine Region umfasst, welche die Objektregion (33) ausschließt.

4. Verfahren nach einem der vorhergehenden Ansprüche, wobei das verarbeitete Bilddatenset (28, 38, 40) in einer semantischen Datenpräsentation repräsentiert wird.

5. Verfahren nach einem der vorhergehenden Ansprüche, wobei das erweiterte Objektmerkmal (19) aus dem verarbeiteten Bilddatenset (28, 38, 40) oder einem verarbeiteten Bilddatenset (28, 38, 40) bestimmt wird, wobei das verarbeitete Bilddatenset (28, 38, 40) durch Verarbeitung der Bilddaten der ersten Region (34) von Interesse erhalten wird, wobei die Verarbeitung eine Normalisierung der Bildintensitäten beinhaltet, die von dem Bilderfassungsmerkmal und/oder dem semantischen Merkmal (20) abhängt.

6. Verfahren nach einem der vorhergehenden Ansprüche, wobei das erweiterte Objektmerkmal (19) aus dem Objektmerkmal (35) und dem semantischen Merkmal (20) basierend auf einer Kombination des Objektmerkmals (35) oder des verarbeiteten Bilddatensets (28, 38, 40) oder eines Merkmals (30) bestimmt wird, das aus dem verarbeiteten Bilddatenset (28, 38, 40) und dem semantischen Merkmal (20) bestimmt wurde.

7. Verfahren nach einem der vorhergehenden Ansprüche, wobei das erweiterte Objektmerkmal (19) aus dem Objektmerkmal (35), dem semantischen Merkmal (20) und einem klinischen Datenmerkmal bestimmt wird.

8. Verfahren nach einem der vorhergehenden Ansprüche, wobei der Schritt der Bestimmung des erweiterten Objektmerkmals (19) aus dem Objektmerkmal (35) und dem semantischen Merkmal (20) unter Verwendung eines Algorithmus durchgeführt wird, der durch maschinelles Lernen trainiert ist, insbesondere durch Deep Learning.

9. Verfahren nach einem der vorhergehenden Ansprüche, wobei das semantische Merkmal (20) eine Objektpositionsinformation umfasst, die eine Objektposition relativ zu einer anatomischen Struktur angibt.

10. Verfahren zum Trainieren eines Algorithmus durch maschinelles Lernen, wobei mehrere Trainingsdatensets zum Trainieren des Algorithmus verwendet werden, wobei jedes Trainingsdatenset ein Bild (14) umfasst, wobei ein jeweiliges erweitertes Objektmerkmal (19) aus dem jeweiligen Bild (14) durch das Verfahren nach einem der vorhergehenden Ansprüche bestimmt und als Eingabe verwendet oder weiter verarbeitet wird, um eine Eingabe in den Algorithmus während des maschinellen Lernens zu generieren.

11. System, umfassend eine Verarbeitungseinheit (44), insbesondere eine Verarbeitungseinheit (44) einer medizinischen Bildgebungsvorrichtung (48), die ausgelegt ist, um das Verfahren nach einem der vorhergehenden Ansprüche durchzuführen.

12. Computerprogramm, das direkt in eine Speichereinheit (46) einer Verarbeitungseinheit (44) geladen werden kann, insbesondere eine Verarbeitungseinheit (44) einer medizinischen Bildgebungsvorrichtung (48), wobei das Computerprogramm Anweisungen zum Durchführen der Schritte des Verfahrens nach einem der Ansprüche 1 bis 10 umfasst, wenn das Programm auf der Verarbeitungseinheit (44) ausgeführt wird.

13. Computerlesbares Speichermedium, das elektronisch lesbare Anweisungen enthält, umfassend das Computerprogramm nach Anspruch 12.

## Revendications

1. Procédé de détermination d'au moins une caractéristique (19) améliorée d'un objet (16, 17, 18, 32) auquel on s'intéresse et qui est dépeint au moins en partie par une image (14) dans une région d'une anatomie d'un patient (43), l'image (14) ayant été donnée par un dispositif (48) d'imagerie,
dans lequel on détermine une caractéristique (35) d'objet respective pour chaque objet (16, 17, 18, 32) et au moins une caractéristique (20) sémantique à partir de la donnée de l'image (14),
dans lequel la caractéristique (35) d'objet dépend d'une information comprise dans la donnée d'image d'une première région (34) à laquelle on s'intéresse, la première région (34) à laquelle on s'intéresse contenant la région (33) de l'objet, et la caractéristique (20) sémantique dépend d'une information comprise dans la donnée d'image d'une deuxième région (37), la deuxième région (37) à laquelle on s'intéresse comprenant une région excluant la région (33) de l'objet, et
dans lequel la caractéristique (20) sémantique comprend une information de contexte anatomique, dans lequel on détermine la caractéristique (19) améliorée de l'objet à partir de la caractéristique (35) d'objet et de la caractéristique (20) sémantique,
dans lequel on détermine la caractéristique (19) améliorée de l'objet à partir de la caractéristique (35) d'objet et de la caractéristique (20) sémantique en traitant la donnée d'image de la première région (34) à laquelle on s'intéresse sur la base de la caractéristique (20) sémantique, pour obtenir un ensemble (28, 38, 40) traité de donnée d'image comprenant la première région (34) à laquelle on s'intéresse et en déterminant la caractéristique (19) améliorée de l'objet à partir de l'ensemble (28, 38, 40) traité de donnée d'image, dans lequel la donnée d'image de la première région (34) à laquelle on s'intéresse dépend d'une géométrie spécifique au patient, qui est décrite par la caractéristique (20) sémantique, dans lequel l'ensemble (28, 38, 40) traité de donnée d'image est obtenu par une transformation de la première région (34) à laquelle on s'intéresse, qui cartographie la géométrie spécifique du patient en un espace (27) géométrique donné normalisé.

2. Procédé suivant la revendication 1, dans lequel on détermine en outre une caractéristique d'acquisition d'image et on détermine la caractéristique (19) améliorée de l'objet à partir de la caractéristique (35) d'objet, de la caractéristique (20) sémantique et de la caractéristique d'acquisition d'image, qui dépend du dispositif (48) d'imagerie et/ou d'un paramètre d'imagerie.

3. Procédé suivant la revendication 2, dans lequel on détermine la caractéristique d'acquisition d'image à partir de la donnée d'image d'une troisième région (25) de l'image (14), la troisième région (25) comprenant une région excluant la région (33) de l'objet.

4. Procédé suivant l'une quelconque des revendications précédentes, dans lequel on représente l'ensemble (28, 38, 40) traité de donnée d'image dans une présentation de donnée sémantique.

5. Procédé suivant l'une quelconque des revendications précédentes, dans lequel on détermine la caractéristique (19) améliorée de l'objet à partir de l'ensemble (28, 38, 40) traité de donnée d'image ou d'un ensemble (28, 38, 40) traité de donnée d'image, dans lequel on obtient l'ensemble (28, 38, 40) traité de donnée d'image en traitant la donnée d'image de la première région (34) à laquelle on s'intéresse, dans lequel le traitement implique une normalisation des intensités d'image, qui dépend de la caractéristique d'acquisition et/ou de la caractéristique (20) sémantique.

6. Procédé suivant l'une quelconque des revendications précédentes, dans lequel on détermine la caractéristique (19) améliorée de l'objet à partir de la caractéristique (35) d'objet et de la caractéristique (20) sémantique sur la base d'une combinaison de la caractéristique (35) d'objet ou de l'ensemble (28, 38, 40) traité de donnée d'image ou d'une caractéristique (30) déterminée à partir de l'ensemble (28, 38, 40) traité de donnée d'image et de la caractéristique (20) sémantique.

7. Procédé suivant l'une quelconque des revendications précédentes, dans lequel on détermine la caractéristique (19) améliorée de l'objet à partir de la caractéristique (35) d'objet, de la caractéristique (20) sémantique et d'une caractéristique de donnée clinique.

8. Procédé suivant l'une quelconque des revendications précédentes, dans lequel on effectue le stade de détermination de la caractéristique (19) améliorée de l'objet à partir de la caractéristique (35) d'objet et de la caractéristique (20) sémantique, en utilisant un algorithme ayant un subi un apprentissage par apprentissage automatique, en particulier un apprentissage profond.

9. Procédé suivant l'une quelconque des revendications précédentes, dans lequel la caractéristique (20) sémantique comprend une information de position de l'objet indiquant une position de l'objet par rapport à une structure anatomique.

10. Procédé d'apprentissage d'un algorithme par apprentissage automatique, dans lequel on utilise de multiples ensembles de donnée d'apprentissage pour faire l'apprentissage de l'algorithme, dans lequel chaque ensemble de donnée d'apprentissage comprend une image (14), dans lequel on détermine une caractéristique (19) respective améliorée de l'objet à partir de l'image (14) respective par le procédé suivant l'une des revendications précédentes, et on l'utilise comme entrée ou on la traite davantage pour créer une entrée à l'algorithme pendant l'apprentissage automatique.

11. Système comprenant une unité (44) de traitement, en particulier une unité (44) de traitement d'un dispositif (48) d'imagerie médicale, configurée pour effectuer le procédé suivant l'une des revendications précédentes.

12. Programme d'ordinateur, qui peut être chargé directement dans une unité (46) de mémoire d'une unité (44) de traitement, en particulier d'une unité (44) de traitement d'un dispositif (48) d'imagerie médicale, le programme d'ordinateur comprenant des instructions pour effectuer les stades du procédé de l'une des revendications 1 à 10, lorsque le programme est exécuté sur l'unité (44) de traitement.

13. Support de mémoire, déchiffrable par ordinateur, contenant des instructions déchiffrables électroniquement, comprenant le programme d'ordinateur suivant la revendication 12.
